# EUROPÄISCHE PATENTSCHRIFT

(11) **EP 0 776 199 B1**
(45) Veröffentlichungstag und Bekanntmachung des Hinweises auf die Patenterteilung: **29.09.1999**
(21) Anmeldenummer: 95929856.3
(22) Anmeldetag: 12.08.1995
(51) Int. Cl.: A61K 9/70

(54) **ESTRADIOL-TTS MIT WASSERBINDENDEN ZUSÄTZEN**
OESTRADIOL TRANSDERMAL THERAPEUTIC SYSTEM COMPRISING HYGROSCOPIC ADDITIVES
SYSTEME THERAPEUTIQUE TRANSDERMIQUE CONTENANT DE L' ESTRADIOL AVEC DES ADDITIFS HYGROSCOPIQUES

(30) Priorität: 20.08.1994 DE 4429667
(43) Veröffentlichungstag der Anmeldung: 04.06.1997
(73) Patentinhaber: LTS LOHMANN Therapie-Systeme GmbH, 56567 Neuwied (DE)
(72) Erfinder: MÜLLER, Walter, D-56564 Neuwied (DE); HORSTMANN, Michael, D-56564 Neuwied (DE)
(74) Vertreter: Flaccus, Rolf-Dieter, Dr.
(86) Internationale Anmeldenummer: EP9503201
(87) Internationale Veröffentlichungsnummer: WO9605814

(56) Entgegenhaltungen:
- EP-A- 0 137 278
- EP-A- 0 421 454
- WO-A-93/02669
- WO-A-93/08795
- WO-A-93/25168
- WO-A-94/23707
- GB-A- 2 158 355
- US-A- 4 855 294
- SUCKER H. ET AL 'Pharmazeutische Technologie' 1991 , GEORG THIEME VERLAG , STUTTGART NEW YORK XP 000189560 189560 siehe Seite 180; Tabelle 1

## Beschreibung

Die Erfindung betrifft ein transdermales therapeutisches System, enthaltend den Wirkstoff Estradiol, mit geschichtetem Aufbau einer wirkstoff- und feuchtigkeitsundurchlässigen Rückschicht, einer wirkstoffhaltigen Matrix und fallweise einer ablösbaren, die Matrix abdeckenden Schutzschicht.

Transdermale therapeutische Systeme (TTS) sind in der Therapie einer Reihe von Erkrankungen bereits im Markt eingeführt.

Auch TTS mit dem Wirkstoff Estradiol sind als Therapeutika für Wechseljahrsbeschwerden, neuerdings auch gegen Osteoporose, im Handel und bewähren sich erfolgreich in der Therapie.

Mit der Bezeichnung "Estradiol" soll im folgenden die wasserfreie Substanz des 17-β-Estradiols verstanden werden.

Ein Nachteil der dem Stand der Technik entsprechenden Systeme ist die nicht ausreichende Permeationsfähigkeit des Wirkstoffs durch die Haut, die auch durch zahlreiche galenische Maßnahmen des TTS-Designs (Einsatz mehrschichtiger Systeme, Verwendung von Steuermembranen, Variation der Wirkstoffkonzentration, Modifikation der Grundpolymeren etc.) nicht über eine gewisse Grenze, den sogenannten "Sättigungsfluß", hinaus steigerbar ist. Diese Feststellung, daß der transdermale Fluß eines Wirkstoffs aus der festen, fein verteilten Phase heraus bereits in den auch heute noch wegweisenden Arbeiten von Higuchi (z.B. T. Higuchi; Physical Chemical Analysis of percutaneous absorption process from creams and ointments. J.Soc. Cosmetic Chem. 11, S. 85.97 (1990))

Die in EP 0 421 454 beschriebenen Systeme enthalten Estradiol in einem Acrylpolymer unter Zusatz von "Kristallisationshemmern" und klebrigmachenden Harzen. Quellstoffe sind zum Schutz vor vorzeitigem Klebkraftverlust enthalten.

Weiterhin gibt es für viele Wirkstoffe die Möglichkeit, dem TTS bei der herstellung sogenannte "Enhancer" zuzusetzen. Es handelt sich hier um in der Regel flüssige Zusatzstoffe, die Resorptionseigenschaften der menschlichen Haut verbessern und damit die Aufnehme des Wirkstoffs aus einer genügend kleinen TTS-Fläche heraus ermöglichen.

Speziell leicht flüchtige Enhancer, wie das zum Beispiel für den Wirkstoff Estradiol verwendete Ethanol, ergeben Probleme infolge einer starken Erweichung der Klebschichten von TTS und machen weitere, raumfordernde Kompartimente im System notwendig, die das TTS unakzeptabel dick werden lassen.

Unter Zusatz weniger flüchtiger, meist aber auch weniger aktiver Enhancer (z.B. Glycerinester, cyclische Amide, Eucalyptol) ist zwar die Herstellung von Matrixsystemen möglich, die Wirkstoff und eine resorptionsverstärkende Komponente in einer oder mehreren Schichten enthalten. Von Nachteil ist jedoch die zu geringe Klebkraft solcher TTS. Die US 4 863 738 stellt ein Beispiel von vielen dar, in denen die Applikation von Wirkstoffen, z.B. Estradiol, zusammen mit einem bestimmten Enhancer (hier Glycerinmonooleat) in einer beliebigen TTS-Matrix in beliebiger Konzentration beansprucht wird.

Aber auch mit einem solchen TTS vom Stand der Technik ist keine befriedigende Therapie möglich, da entweder die ausgewählten Enhancer zu schlecht hautverträglich sind, oder die Systeme wegen des zu geringen Wirkstoffflusses durch die Haut unakzeptabel große Flächen aufweisen.

Eine andere (theoretische) Möglichkeit, den Wirkstofffluß durch die Haut zu steigern, besteht darin, mehr Wirkstoff im TTS molekulardispers, d.h. kristallfrei zu lösen als der Sättigungslöslichkeit entspricht. Mit dem Ausmaß der Übersättigung solcher Systeme steigt im gleichen Maße auch die Permeationsrate durch die Haut an. Solche physikalischen Zustände sind jedoch thermodynamisch instabil, weshalb diese Arzneiformen nicht lagerfähig sind. Es findet innerhalb von Monaten und spätestens Jahren eine spontane, unvorhersehbare Ausfällung von Wirkstoff statt, so daß die Flußrate durch die Haut nach und nach auf Sättigungsflußniveau abfällt und damit je nach Ausgangskonzentration ein großer Teil der initial vorhandenen therapeutischen Aktivität verloren geht.

Dieser bei Lagerung stattfindende Vorgang ist durch besondere physikochemische Eigenheiten des Estradiols bedingt.

Estradiol liegt bei Raumtemperatur und gewöhnlicher relativer Luftfeuchte (20-60% relativer Feuchte) nicht in einer der beiden bekannten kristallwasserfreien Modifikationen (I und II) vor, sondern als Semihydrat (Busetti, Acta Cryt. 1972, B28, 560). Wegen der schichtförmigen, über Wasserstoffbrücken stabilisierten Struktur und der Diffusionsdichtigkeit des Kristallverbundes läßt sich das Hydrat kurzzeitig unzersetzt bis zu Temperaturen von ca. 170°C erhitzen (Kuhnert-Brandstätter u. Winkler (1976) Scientia Pharmaceutica 44(3), 177-190). Durch Mikronisieren kann Estradiol-semihydrat jedoch auf dem Wege der Kristalloberflächenvergrößerung bereits bei ca. 120°C quantitiv in die wasserfreie Form umgewandelt werden. Nach eigenen Beobachtungen findet bei langsamem Erwärmen (0,2-1 K/min) und besonders feiner Substanz die Umwandlung bereits bei etwa 90°C statt.

Andererseits weist Estradiol mit geringerer werdendem Wasserdampfpartialdruck höhere Löslichkeiten in einigen Polymeren, speziell Polyacrylaten auf. Da mit höherer Konzentration unter sonst gleichen Bedingungen der Diffusionsfluß durch die Haut nach dem Fickschen Gesetz zunimmt, ist eine solche Konzentrationssteigerung bei transdermalen therapeutischen Systemen sehr erwünscht. Jedoch reicht bereits das mit dem Estradiol-semihydrat eingebrachte Wasser aus, um nach und nach ein erneutes Auskristallisieren als Estradiol-semihydrat aus der Lösung zu bewirken (Kuhnert-Brandstätter u. Winkler (1976) Scientia Pharmaceutica 44(3), 177-190). Beim Auskristallisieren fällt mit geringer werdender Konzentration die Flußrate aus dem System an die Haut stark ab.

Dementsprechend sind transdermale Systeme bekannt, welche durch genaue Konzentrationseinstellung unter der Sättigungslöslichkeit des Estradiol-Anhydrates (DE-PS 42 37 453) oder durch Verwendung teilweise ungelösten, dispergierten Estradiol-Anhydrates (DE-PS 42 23 360) eine pharmakotherapeutisch befriedigende Lösung anbieten. Auch bei Berücksichtigung dieses aktuellen Standes der Technik ist es wichtig, während Herstellung und Lagerung eines Estradiol-TTS eine ausreichend niedrige Luftfeuchtigkeit aufrechtzuerhalten, damit großflächige Ausfällung des schwerlöslichen Estradiol-semihydrates vermieden wird.

Hierzu kann grundsätzlich eine Verpackung mit geringer Wasserdampfdurchlässigkeit dienen. Bei den geringen Mengen Estradiol, die in heutigen TTS enthalten sind, reichen jedoch sehr geringe Feuchtigkeitsmengen zur Ausfällung des Estradiol-semihydrates aus. Sind beispielsweise 2 mg Estradiol (wasserfrei) in einem TTS gelöst vorhanden, ist bereits eine (sich aus dem Molekulargewichtsverhältnissen) errechnende Menge von nur 66,1 µg Wasser zur vollständigen Ausfällung in der Lage. Es ist dementsprechend mit konventionellen Packmitteln sehr schwierig, über Lagerzeiten von mehreren Jahren den Zutritt solch geringer Feuchtigkeitsmengen auszuschließen.

Aufgabe dieser Erfindung ist es daher, ein transdermales therapeutisches System mit Estradiol anzugeben, das in die wirkstoffhaltigen Schichten selbst eingearbeitet einen über längere Zeit wirksamen Schutz vor Ausfällung des Estradiol-semihydrates enthält und ein Auskristallisieren zum Estradiol-semihydrat verhindert. Dieses Ziel wird durch das Kennzeichen des Anspruchs 1 erreicht.

Glycerin ist eine sehr polare Verbindung und in jedem Verhältnis mit Wasser mischbar. Handelsübliche Lieferformen enthalten meist bis über 10 % Wasser. Wasserfreies Glycerin ist stark hygroskopisch und kann als wasserentziehendes Mittel unter bestimmten Bedingungen eingesetzt werden. Experimentell kann gezeigt werden, daß es in wasserfreier Form in der Lage ist, dem Estradiol-semihydrat Kristallwasser zu entziehen. Unter "wasserfrei" bzw. "im wesentlichen wasserfrei" wird dabei ein Wassergehalt von weniger als 1 % verstanden. Wird Estradiol-semihydrat etwa 24 Stunden bei Raumtemperatur in wasserfreiem Glycerin gerührt, wandeln sich die flächigen Kristalle des Semihydrates in dünne Nadeln um. Bei diesen Nadeln handelt es sich entweder um das wasserfreie Estradiol oder um ein Estradiol-Glycerin-Solvat. Schon der Zusatz von 2% Wasser zu dem für diesen Versuch verwendeten Glycerin verhindert diese Reaktion. Damit ist ganz klar demonstriert, daß die bei der Reaktion mit wasserfreiem Glycerin entstehende Spezies kein Wasser enthält.

Zwar wird Glycerin neben zahlreichen anderen Stoffen in der WO 93/08795 als Penetrationsverstärker für transdermale Systeme mit unterschiedlichen Wirkstoffen - u.a. auch mit Estradiol - genannt, jedoch konnte daraus kein Hinweis auf die besondere Nützlichkeit von praktisch wasserfreiem Glycerin in den genannten Mengen speziell für Estradiol enthaltende transdermale therapeutische Systeme hergeleitet werden.

Die vorstehend beschriebene erfindungsgemäße Ausbildung eines TTS kann auf unterschiedliche Weise realisiert werden. Die einfachste Form ist ein einschichtiges Matrixsystem, dessen Matrix von ihrer Funktion her gleichzeitig haftklebend ist und damit eine spezielle Klebschicht überflüssig macht. Durch das in der Matrix dispergierte Glycerin wird über die Lagerdauer eine derart niedrige Gleichgewichtsfeuchte eingestellt, daß eine Fällung von Estradiol-semihydrat unmöglich wird.

Wenn die Klebkraft dieser Schicht nicht ausreichend ist oder wenn der direkte Hautkontakt dieser Schicht vermieden werden soll, kann die Matrix mit einer speziellen Hautkleberschicht kaschiert werden.

Wird zwischen einer solchen Matrix, enthaltend Estradiol und wasserbindendes dispergiertes Glycerin, und Klebschicht eine für Estradiol schwer durchlässige Membran eingebracht, erreicht man eine stärker vom Pflaster als von der Haut gesteuerte Wirkstoffabgabe.

Als Grundmaterial können neben den weitverbreiteten und für Estradiol gut einsetzbaren Acrylsäurecopolymeren auch andere Polymere, wie Polyisobutylen, Polyvinylacetat und Copolymere, Synthesekautschuk, Blockpolymere aus Styrol und Isopren bzw. Styrol und Butadien, und Silicone verwendet werden.

Kennzeichnend für erfindungsgemäße transdermale therapeutische Systeme ist in jedem Fall die Anwesenheit von dispergiertem, im wesentlichen wasserfreiem Glycerin. Dabei ist der genaue Anteil des Glycerins im System so hoch zu wählen, daß einerseits die Löslichkeit des Glycerins im System überschritten ist und es in kleinen Tröpfchen dispergiert als separate Phase vorliegt, und andererseits die Gesamtfeuchtigkeitsbindung im System ausreichend ist. Als minimal kann ein Zusatz in der Größenordnung von 2 Prozent gelten; die Obergrenze wird durch mechanische Größen wie Fließfähigkeit der Matrix, Klebkraft und Verarbeitbarkeit bestimmt; in der Regel wird ein Anteil von 10 bis 50 Gewichtsprozenten, bevorzugt zwischen 10 und 35 Gewichtsprozenten, angestrebt.

### Beispiele:

### Beispiel 1

2,0 g 17-β-Estradiol-semihydrat, mikronisiert werden mit
2,75 g wasserfreiem Glycerin,
25 g einer Acrylestercopolymerlösung (Festkörpergehalt 42%) und
5,9 g Kolophonium-Glycerolesterderivat (Staybelite Ester 5E, Fa. Hercules)
   gemischt und anschließend auf eine 100 µm dicke, silikonisierte Polyesterfolie so beschichtet, daß ein Beschichtungsgewicht von 120 g/m² resultiert.
   Der Ausstrich wird je 10 Minuten lang bei 25 °C, bei 50 °C, bei 80 °C und bei 95 °C getrocknet. Sofort wird 10 µm dicke Polyesterfolie luftblasenfrei unter Walzendruck auf die getrocknete Schicht aufgelegt (zukaschiert).
   Durch Stanzung mit Henkellocheisen werden transdermale therapeutische Systeme von 16 cm² erhalten, die sofort in wasserdampfdicht heißsiegelbare Beutel verpackt werden.

### Beispiel 2:

2,0 g 17-β-Estradiol-semihydrat, mikronisiert,
60,0 g Cariflex TR 1107® (Styrol-Isopren-Blockpolymer),
120,0 g Staybelite Ester 5E (thermoplastisches Esterharz aus Kolophoniumderivaten),
50,0 g dickflüssiges Paraffin,
50,0 g wasserfreies Glycerin
   werden in einem evakuierbaren Kneter bei 130 °C aufgeschmolzen und durch Kneten innerhalb von 10 Stunden in eine äußerlich homogene Form gebracht.
   Die Schmelze wird auf 120 °C abgekühlt und anschließend auf einer kontinuierlich arbeitenden Beschichtungsanlage auf 100 µm dicke, silikonisierte Polyesterfolie so beschichtet, daß ein Flächengewicht von 200 g/m² resultiert.
   Anschließend wird eine 15 µm dicke Polyesterfolie luftblasenfrei unter Walzendruck auf die noch warme Schicht aufgelegt (zukaschiert).

Durch die Stanzung mit Henkellocheisen werden transdermale therapeutische Systeme von 16 cm² erhalten.

## Patentansprüche

1. Transdermales therapeutisches System, enthaltend den Wirkstoff Estradiol, mit geschichtetem Aufbau einer wirkstoff- und feuchtigkeitsundurchlässigen Rückschicht, einer wirkstoffhaltigen Matrix und fallweise einer ablösbaren, die Matrix abdeckenden Schutzschicht, dadurch gekennzeichnet, daß die Matrix 10 - 35 % (G/G) Glycerin mit einem Wassergehalt ≤ 1 % enthält.

2. Transdermales therapeutisches System nach Anspruch 1, dadurch gekennzeichnet, daß die Matrix aus mehreren Schichten besteht und mindestens eine Schicht Glycerin enthält.

3. Transdermales therapeutisches System nach Anspruch 1 oder 2, dadurch gekennzeichnet, daß das in der Matrix enthaltende Estradiol als Dispersion eines wasserfreien Kristallisats vorliegt.

4. Transdermales, therapeutisches System nach einem oder mehreren der Ansprüche 1 bis 3, dadurch gekennzeichnet, daß das Grundmateril der Matrix oder einer ihrer Schichten ein Acrylsäureestercopolymer ist.

5. Transdermales therapeutisches System nach Anspruch 4, dadurch gekennzeichnet, daß das Acrylsäureestercopolymer eine Löslichkeit für das Kristallisat des Estradiols zwischen 0,4 und 3,0 % aufweist.

## Claims

1. A transdermal therapeutic system comprising the active substance estradiol and having a layered structure of a backing layer which is impermeable to active substances and moisture, an active substance-containing matrix, and, in case of need, a removable protective layer covering the matrix, characterized in that the matrix comprises 10 - 35%-wt. of glycerol having a water content ≤ 1%.

2. The transdermal therapeutic system according to claim 1 characterized in that the matrix consists of several layers and that at least one layer comprises glycerol.

3. The transdermal therapeutic system according to claim 1 or 2 characterized in that the estradiol comprised in the matrix is present as dispersion of an anhydrous crystallizate.

4. The transdermal therapeutic system according to one or several of claims 1 to 3 characterized in that the base material of the matrix or of one of its layers is an acrylic-acid ester copolymer.

5. The transdermal therapeutic system according to claim 4 characterized in that the acrylic-acid ester copolymer has a solubility for the crystallizate of the estradiol of between 0.4 and 3.0%.

## Revendications

1. Système thérapeutique transdermique, contenant de l'estradiol comme substance active, présentant une structure à couches constituée par un revers imperméable à la substance active et à l'humidité, une matrice contenant la substance active et, le cas échéant, une couche de protection détachable, recouvrant la matrice, caractérisé en ce que la matrice contient 10 à 35 % (en poids) de glycérine, présentant une teneur en eau ≤ à 1 %.

2. Système thérapeutique transdermique selon la revendication 1, caractérisé en ce que la matrice est constituée par plusieurs couches et contient au moins une couche de glycérine.

3. Système thérapeutique transdermique selon la revendication 1 ou 2, caractérisé en ce que l'estradiol contenu dans la matrice se présente sous forme d'une dispersion d'un produit de cristallisation exempt d'eau.

4. Système thérapeutique transdermique selon une quelconque des revendications 1 à 3, caractérisé en ce que le matériau de base de la matrice ou d'une de ses couches est un copolymère d'un ester de l'acide acrylique.

5. Système thérapeutique transdermique selon la revendication 4, caractérisé en ce que le copolymère d'un ester de l'acide acrylique présente une solubilité pour le produit de cristallisation de l'estradiol comprise entre 0,4 et 3,0 %.
